# EUROPEAN PATENT APPLICATION

(11) **EP 4 563 594 A1**
(43) Date of publication of application: **04.06.2025**
(21) Application number: 23383220.3
(22) Date of filing: 28.11.2023
(51) Int. Cl.: C07K 16/24, A61K 31/00, A61P 35/00, A61K 39/00

(54) **CXCL8 INHIBITORS FOR USE IN THE TREATMENT OF TUMORS WITH LOW STROMAL CAV1 LEVELS**

(71) Applicant: Dompe' Farmaceutici SpA, 20121 Milano (IT); Centro Nacional de Investigaciones Cardiovasculares Carlos III (F.S.P.), 28029 Madrid (ES)
(72) Inventor: Brandolini, Laura, 67100 L'Aquila (IT); Giorgio, Cristina, 67100 L'Aquila (IT); del Pozo Barriuso, Miguel Ángel, 28029 Madrid (ES); Díez Sánchez, Alberto, 7030 Trondheim (NO); Sánchez Álvarez, Miguel, 28029 Madrid (ES)
(74) Representative: Dompé farmaceutici Spa

(57) **Abstract**

The invention relates to CXCL8 inhibitors for use in the treatment of a tumor with low stromal CAV1 levels and for use in the prevention and/or treatment of a metastasis of a tumor with low stromal CAV1 levels.

## Description

### TECHNICAL FIELD

The invention relates to CXCL8 inhibitors for use in the treatment of tumors with low stromal CAV1 levels.

### BACKGROUND OF THE INVENTION

The tumor microenvironment (TME) or tumor stroma, which mainly consists of fibroblasts, immune cells, extracellular matrix, and the vasculature that surround cancer cells, has been described to be able to influence disease progression (Quail, D. F. et al., Nat Med 19, 1423-1437 (2013)). Tumor stroma has resulted to be a key driver of tumor progression and metastasis, as it defines the microenvironment with which tumor cells interact and adapt to, in order to survive, thrive and metastasize.

Cancer Associated Fibroblasts (CAFs) found in the tumor microenvironment (TME) are key regulators of tumor growth, aggressiveness and therapeutic resistance, but how their functional state determines their impact on disease progression is poorly understood.

Caveolin-1 (CAV1) is a biomarker of lipid microdomains (including lipid rafts and caveolae) on the cell membrane, and is involved in the generation and function of caveolae (Sotgia F., et al., Annu Rev Pathol: Mechanisms of Disease, 2012; 7: 423-467), such as caveolae-mediated endocytosis and transcytosis (Cheng J.P., et al., Trends Cell Biol. 2016; 26: 177-189), cholesterol transport between cytoplasm and membrane to maintain lipid homeostasis (Li X.A., et al., Trends Cardiovasc Med. 2005; 15: 92-96). Moreover, caveolin-1 mediated signal transduction is also reported to affect many bio-effects, such as cell cycle regulation (Galbiati F., et al., Mol Biol Cell. 2001; 12: 2229-2244), proliferation, invasion and cell death (Engelman J.A., et al., J Biol Chem. 1997; 272: 16374-16381; Torres V.A., et al., J Cell Sci. 2006; 119: 1812-1823; Xu L., et al., Oncol Rep. 2014; 32: 318-324; Meyer C., et al., Cell Death Dis. 2013; 4 e466-e66).

Clinically, it has been observed that the downregulation or deletion of caveolin-1 on stromal CAFs leads to poor tumor prognosis.

Tumors bearing low CAV1-CAFs display an aberrant network of poorly perfused, leaky capillaries, display hypoxia and fibrosis, and show increased invasiveness and poor survival upon resection.

Previous studies support that CAV1 levels in CAFs influence breast cancer growth and metastasis, but the molecular mechanisms underlying these effects and their contextuality remain incompletely understood.

A major portion of the breast tumor stroma is occupied by CAFs (Kalluri, R. et al., Fibroblasts in cancer. Nat. Rev. Cancer 6, (2006); Petrova, V., et al., Oncogenesis 7, 10 (2018)), which through the secretion of growth factors and chemokines, as well as the production and modification of extracellular matrix (ECM), have been demonstrated to contribute to the core and emergent hallmarks of cancer (Hanahan, D. et al., Cell 144, 646-674 (2011)). Among these hallmarks, CAFs have been shown to sustain proliferative signalling, induce angiogenesis, activate invasion and metastasis, reprogram energy metabolism, and help evading tumor immune destruction (Hanahan, D. et al., Cancer Cell 21, 309-322 (2012); Petrova, V., et al., Oncogenesis 7, 10 (2018); Tao, L., Huang, et al., Oncol. Lett. 14, 2611-2620 (2017)).

CAFs have also been associated with the increased interstitial fluid pressure and the faulty vascular network present in solid tumors, thus indirectly decreasing the efficiency of drug delivery to cancer cells. Through this and the above-mentioned hallmarks, CAFs have emerged as key players in promoting cancer therapy resistance (Kalluri, R., Nat. Rev. Cancer 16, 582-598 (2016)).

In various cancer types, chemo-drug resistance is related to the overexpression of CAV1.

For example, CAV1 is associated with drug resistance to colon, ovarian, gastric, lung and breast cancer.

For breast cancer subtypes without a drug specifically approved for its treatment, as in the case of Triple Negative Breast Cancer (TNBC) (Gadi, V. K. et al., J. Oncol. Pract. 13, 293-300 (2017)), the efficient delivery of common chemotherapeutics to the tumor constitutes a chance for improvement in treatment success. This enhanced drug delivery can be achieved through tumor vessel normalization (Goel, S., Wong et al., Cold Spring Harb. Perspect. Med. 2, a006486 (2012)).

Given the importance of CAFs in the modulation of breast cancer growth, metastasis and response to therapy, understanding the molecular mechanisms driving CAFs behavior and finding CAFs markers for breast cancer patient stratification has been the focus of research recently (Finak, G. et al., Nat. Med. 14, 518-527 (2008); Winslow, S., et al., Breast Cancer Res. 17, 23 (2015); Wennmalm, K., et al., Nat. Med. 15, 237-238 (2009)).

Amidst these biomarkers, changes in the expression levels of CAV1 in CAFs have been associated with the metabolic reprogramming of the tumor (Migneco, G. et al., Cell Cycle 9, 2412-2422 (2010)) and the modification of the ECM architecture (Goetz, J. G. et al. Biomechanical remodeling of the microenvironment by stromal caveolin-1 favors tumor invasion and metastasis. Cell (2011) Jul 8; 146(1): 148-63), which in turn modulate breast cancer cell growth and metastasis.

CAV1 is a scaffolding protein with multiple binding partners that is associated with cell surface caveolae, the regulation of lipid raft domains and lipid trafficking (Parton, R. G. et al., Nat. Rev. Mol. Cell Biol. 14, 98-112 (2013)).

CAV1 regulates multiple cancer-associated processes including cellular transformation, tumor growth, cell migration and metastasis, cell death and survival, multidrug resistance, and angiogenesis (Goetz, J. G., et al., Cancer Metastasis Rev. 27, 715-735 (2008)).

However, low levels of CAV1 in the stroma have been reported to impact both positively and negatively on various aspects of tumor progression and while described to function as a tumor suppressor, it has also been identified as a poor prognostic factor in various human cancers (Martinez-Outschoorn, U. E., et al., Nat. Rev. Cancer 15, 225-237 (2015)). Although multiple mechanisms have been proposed to explain the deregulation of CAV1 levels in CAFs (Chen, D. et al., Oncol Lett 8, 1409-1421 (2014)), the reason behind these changes still remains a puzzle (Mao, Y., et al., Cancer Metastasis Rev. 32, 303-15). CAV1 thus appears to have a complex role in tumor stroma.

Given the poor prognosis of tumors with low stromal CAV1 levels, there is still a high need for effective and safe therapies.

Different CXCL8 inhibitors have been developed and are well known to the skilled person.

WO2000/024710 discloses N-(2-aryl-propionyl)-sulfonamides, having inhibitory activity of neutrophils chemotaxis and degranulation induced by interleukin-8, and their use in the prevention and treatment of tissue damage due to the exacerbate recruitment of polymorphonuclear neutrophils (leukocytes PMN) at the inflammatory sites, in particular in the treatment of psoriasis, rheumatoid arthritis, ulcerative colitis, acute respiratory insufficiency, idiopathic fibrosis, glomerulonephritis.

WO2005/090295 discloses (R)-2-[4-(trifluoromethanesulfonyloxy)phenyl]propionic acid derivatives, which are used as inhibitors of the chemotaxis of polymorphonucleate and mononucleate cells, particularly in the treatment of neutrophils-dependent pathologies. The use of said compounds in the treatment of psoriasis, ulcerative colitis, melanoma, angiogenesis, chronic obstructive pulmonary disease (COPD), bullous pemphigo, rheumatoid arthritis, idiopathic fibrosis, glomerulonephritis and in the prevention and treatment of damages caused by ischemia and reperfusion is also disclosed.

WO2010/031835 discloses 2-aryl-propionic acids and derivatives, substituted in position 4 with 2-amino-heterocycles, as CXCL8-induced chemotaxis inhibitors, useful in the prevention and treatment of tissue damage due to the exacerbated recruitment of polymorphonucleated neutrophils (PMN leukocytes) at inflammation sites. The use of said compounds in the treatment of transient cerebral ischemia, damages caused by ischemia and reperfusion, bullous pemphigo, rheumatoid arthritis, idiopathic fibrosis and glomerulonephritis is also disclosed.

The present invention is aimed at providing an effective treatment of tumors with low stromal CAV1 levels.

### SUMMARY OF THE INVENTION

The invention is directed to a CXCL8 inhibitor for use in the treatment of a tumor with low stromal CAV1 levels.

The invention is also directed to a pharmaceutical composition comprising at least one CXCL8 inhibitor and at least one pharmaceutically acceptable excipient or carrier for use in the treatment of a tumor with low stromal CAV1 levels.

The invention is also directed to a method of treating a tumor with low stromal CAV1 levels, which comprises administering an effective amount of one or more CXCL8 inhibitors to a subject in need thereof.

The invention is also directed to the use of a CXCL8 inhibitor in the manufacture of a medicament for the treatment of a tumor with low stromal CAV1 levels.

### BRIEF DESCRIPTION OF FIGURES

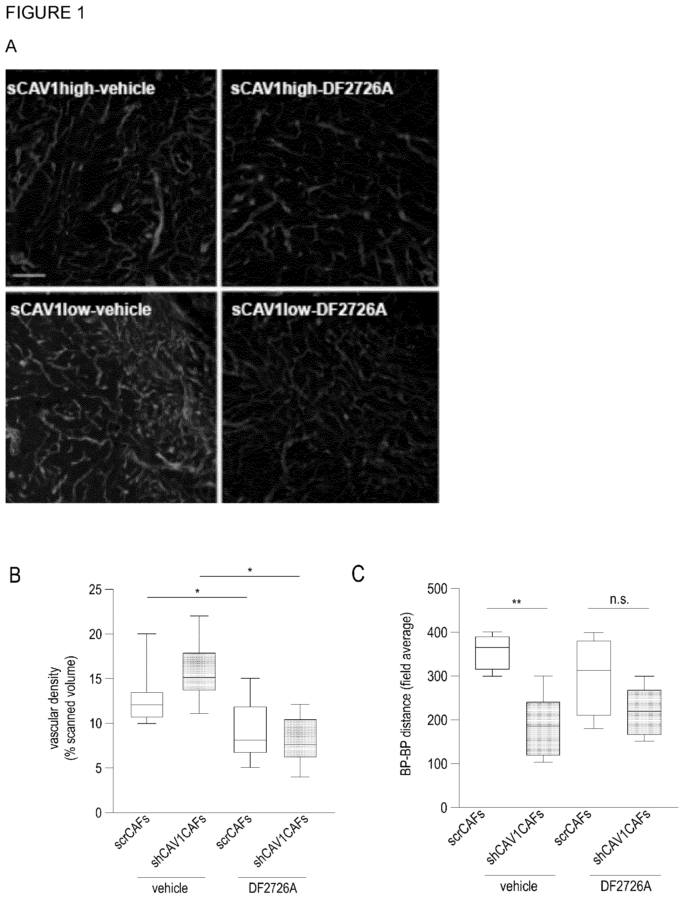

**Figure 1****:** panel A shows representative images of anti-PECAM1 -stained tumor sections across indicated treatments (DF2726A is an inhibitor of CXCL8 signalling via CXCR1/2 receptor inhibition) and stromal genotypes. Panel B and C show plots of indicated parameters (in panel B: vascular density; in panel C: contiguous branchpoints distance (BP-BP)) as assessed by quantitative image analysis across 3 independent fields per primary tumor (n individuals: 10 per genotype and condition).

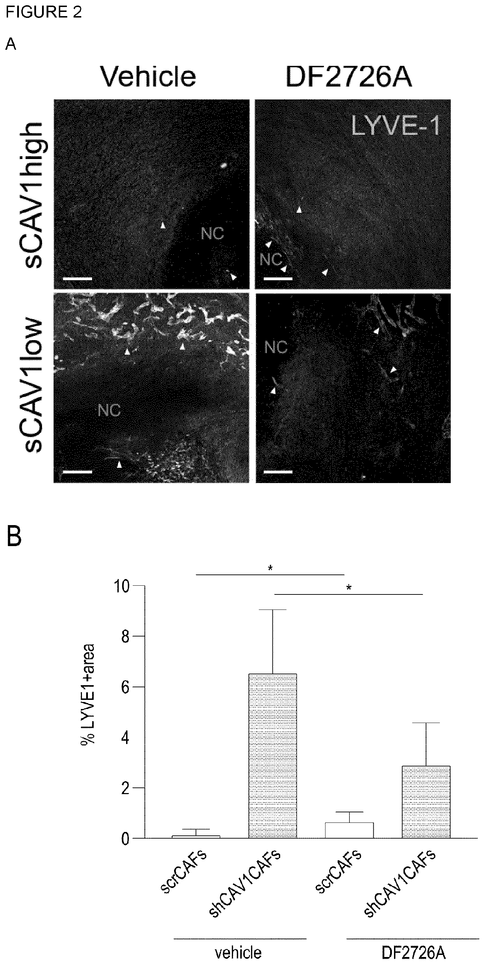

**Figure 2****:** panel A shows representative images of anti-LYVE1-stained tumor sections across indicated treatments (DF2726A is an inhibitor of CXCL8 signalling via CXCR1/2 receptor inhibition) and stromal genotypes. LYVE1-positive structures (lymphatic vessels and sprouts; arrowheads) around the necrotic core of the primary tumor mass (NC) are indicated. Panel B shows plots representing the percentage of the LYVE1-+area (across 1-4x whole image average) across 3 independent fields per primary tumor (n individuals: 10 per genotype and condition).

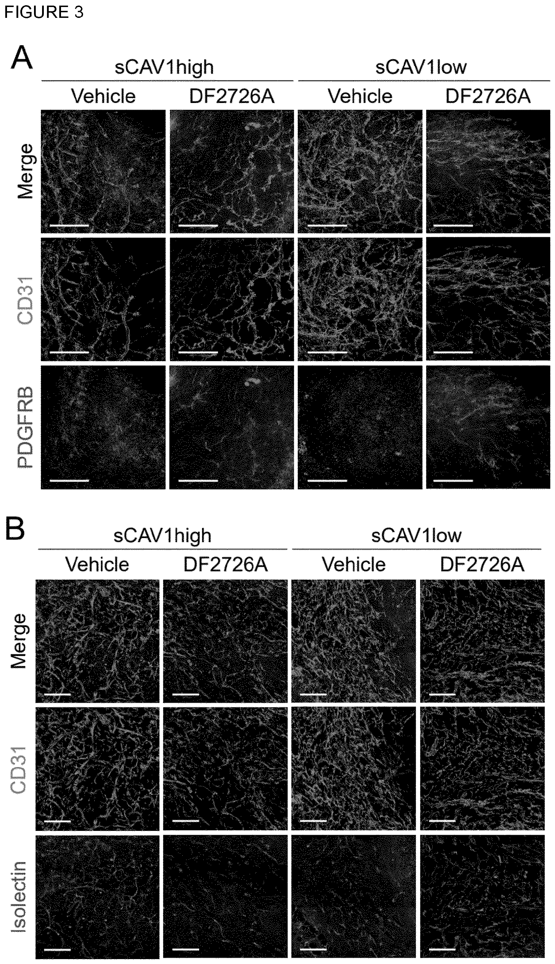

**Figure 3****:** panel A shows representative tumor xenograft sections depicting distinct blood vessel densities and pericyte coverage. Blood vessels are stained using the endothelial cell marker CD31 and pericytes using PDGFRB. Increased pericyte coverage is associated with mature and functional blood vessels; n=3 mice/vehicle group & n=5 mice/treated group. Scale bar: 200µm. Panel B shows representative tumor xenograft sections depicting differential perfusion upon vehicle or CXCL8 inhibitor (DF2726A is a CXCR1/2 inhibitor) treatment. Blood vessels are revealed by CD31 and isolectin staining demarks properly perfused capillaries; n=8 mice/ vehicle group & n=10 mice/ treated group. Data is derived from 2 independent experiments. Scale bar: 200µm.

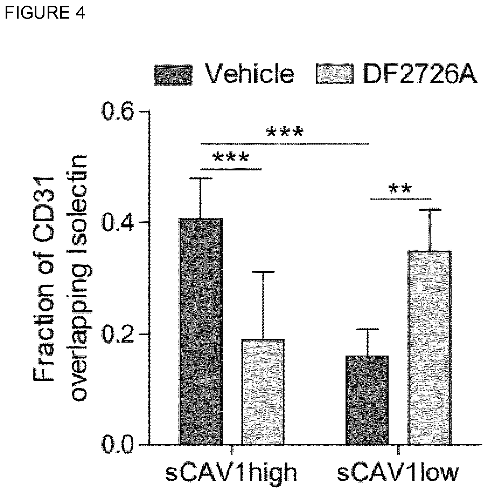

**Figure 4****:** shows a panel with the quantification of isolectin and CD31 positive tumor blood vessels for proper perfusion determination. Data are presented as mean ± SD; n=8 mice/ vehicle group & n=10 mice/ treated group. Data is derived from 2 independent experiments, **P<0.01,***P<0.001, (One-way ANOVA, Tukey's multiple comparison test).

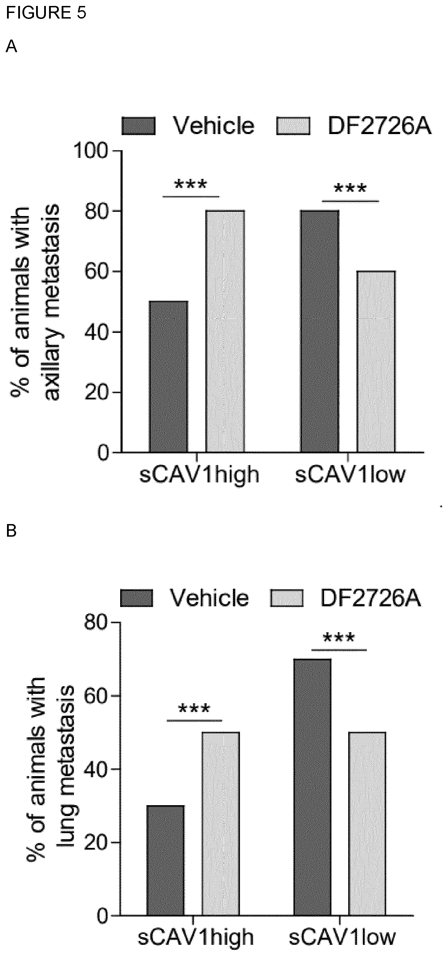

**Figure 5****:** shows incidence of axillary (panel A) or lung (panel B) metastasis across indicated treatment conditions, expressed as % of animals for which metastatic foci were detected upon anatomopathological inspection. Quantitation is shown for n=10 animals, except for sCAV1 high-vehicle condition (n=9).

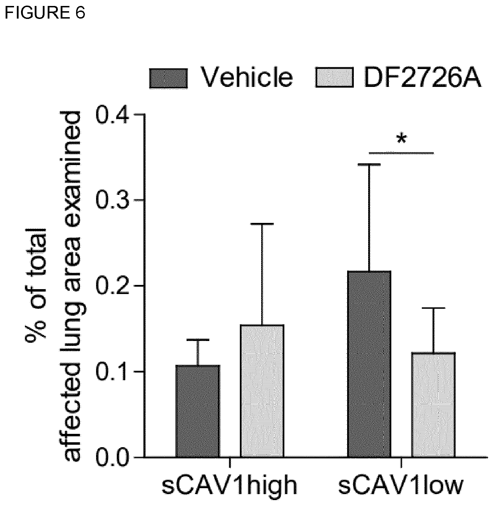

**Figure 6****:** shows the relative extension of lung metastases expressed as % of total lung inspected area. Quantitation is shown for n=10 animals, except for sCAV1 high-vehicle condition (n=9). Statistical significance (unpaired t-Student test) of indicated comparisons is highlighted as * P<0.05.

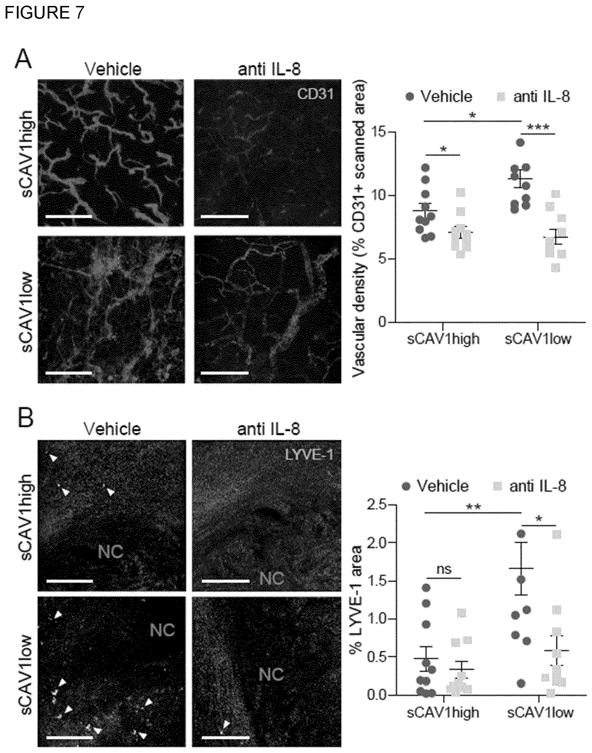

**Figure 7****:** left side of panel A shows representative images of tumor sections stained using the endothelial cell marker CD31 across indicated treatments and stromal genotypes; right side of panel A shows plot of vascular density as assessed by quantitative image analysis across 3 independent fields per primary tumor (*n* individuals: 10 per genotype and condition). Left side of panel B shows representative images of anti-LYVE1-stained tumor sections across indicated treatments and stromal genotypes. LYVE1-positive structures (lymphatic vessels and sprouts; arrowheads) around the necrotic core of the primary tumor mass (NC) are indicated. Right side of panel B shows plots representing the percentage of the LYVE1-+area (across 1-4x whole image average) across 3 independent fields per primary tumor (*n* individuals: 10 per genotype and condition). Statistical significance (unpaired t-Student test) of indicated comparisons is highlighted as * P<0.05, ** P<0.01 and *** P<0.001.

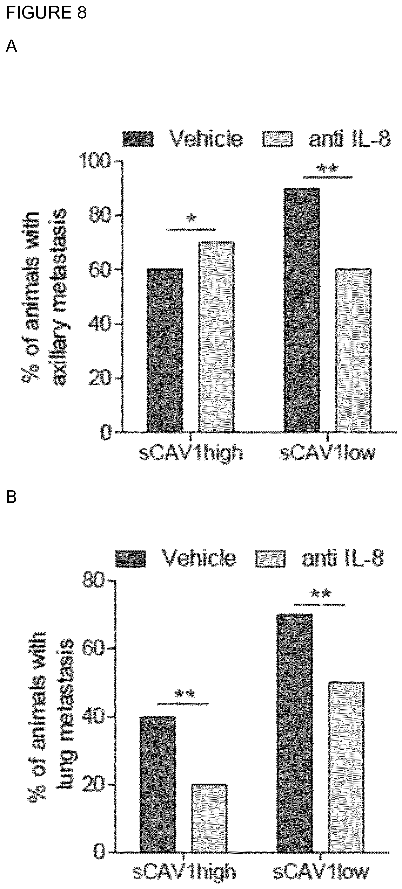

**Figure 8****:** shows incidence of axillary (panel A) or lung (panel B) metastasis across indicated treatment conditions, expressed as % of animals for which metastatic foci were detected upon anatomopathological inspection. Quantitation is shown for n=10 animals, except for sCAV1high-vehicle condition (n=9). Statistical significance was assessed using Barnard's Exact test (* P<0.05 and ** P<0.01).

### DETAILED DESCRIPTION OF THE INVENTION

It has been surprisingly found that CXCL8 inhibitors are effective in the treatment of tumors with low stromal CAV1 levels and in the prevention and/or treatment of metastases of tumors with low stromal CAV1 levels.

Accordingly, the invention is directed to a CXCL8 inhibitor for use in the treatment of a tumor with low stromal CAV1 levels.

According to a preferred embodiment, the invention is directed to a CXCL8 inhibitor for use in the prevention and/or treatment of a metastasis of a tumor with low stromal CAV1 levels.

According to a preferred embodiment, said metastasis of a tumor with low stromal CAV1 levels is a metastasis of breast cancer with low stromal CAV1 levels, preferably triple negative breast cancer with low stromal CAV1 levels.

According to a preferred embodiment, said metastasis of a tumor with low stromal CAV1 levels is a lung metastasis, preferably a lung metastasis of breast cancer with low stromal CAV1 levels, preferably a lung metastasis of triple negative breast cancer with low stromal CAV1 levels.

According to another preferred embodiment, said metastasis of a tumor with low stromal CAV1 levels is an axillary metastasis, preferably an axillary metastasis of breast cancer with low stromal CAV1 levels, preferably an axillary metastasis of triple negative breast cancer with low stromal CAV1 levels.

The term "CXCL8 inhibitor" in accordance with the present invention means any compound able to inhibit the biological activity of CXCL8.

Methods for determining the inhibition of the biological activity of CXCL8 and for classifying a compound as "CXCL8 inhibitor" are known in the art and are described, for example, in Moriconi et al., J. Med. Chem. 2007, 50, 3984-4002, and in Brandolini et al., Scientific Reports (2019) 9:11729.

Preferably, a CXCL8 inhibitor according to the present invention is a CXCL8 receptor(s) inhibitor.

Preferably said CXCL8 receptor(s) inhibitor is a CXCR1- or a CXCR1/2-inhibitor, which inhibits the activity of CXCL8 mediated by the CXCR1 receptor or mediated by both the CXCR1 and CXCR2 receptors.

Said CXCL8 receptor(s) inhibitor preferably inhibits the binding of CXCL8 to the CXCR1 receptor (CXCR1 receptor inhibitor) or to both the CXCR1 and CXCR2 receptors (dual CXCR1 and CXCR2 receptor inhibitor), or prevents or blocks the intracellular signaling activated by the binding of CXCL8 to the CXCR1 receptor (CXCR1 receptor inhibitor) or to both the CXCR1 and CXCR2 receptors (dual CXCR1 and CXCR2 receptor inhibitor).

According to a preferred embodiment, the CXCL8 receptor(s) inhibitor is an antagonist of the CXCR1 receptor or an antagonist of both the CXCR1 and CXCR2 receptors.

According to another preferred embodiment, the CXCL8 receptor(s) inhibitor is an allosteric inhibitor or an orthosteric antagonist of the CXCR1 receptor or of both the CXCR1 and CXCR2 receptors.

Alternatively, the CXCL8 inhibitor preferably binds to CXCL8, thus preventing its binding to its receptors.

Said CXCL8 receptor(s) inhibitor is preferably able to inhibit in an in-vitro assay at least 60%, preferably at least 70%, more preferably at least 80%, even more preferably at least 90% of PMNs chemotaxis induced by 1 nM CXCL8 at a concentration equal or below 500 nM, preferably below 100 nM.

More preferably, the CXCL8 receptor(s) inhibitor according to the invention has an IC₅₀ value towards the CXCR1 receptor in the low nanomolar range, preferably lower than 10 nanomolar, more preferably in the range 0.02-5 nanomolar.

According to a further preferred embodiment, said CXCL8 inhibitor is selected from small molecules, peptides and antibodies, more preferably it is a small molecule.

The term "small molecule" refers to an organic compound having a molecular weight of 900 Daltons or lower.

CXCL8 inhibitors, in particular CXCL8 receptor(s) inhibitors, as defined above, are well known in the art.

To date, several CXCL8 inhibitors, such as small molecules, peptides and antibodies, have been disclosed, many of which are currently undergoing clinical trials or are used in therapy (Jie Jack, Expert Opinion Ther. Patents, 2001, 11(12), Chao J. et al., Bioorganic & Medicinal Chemistry Letters 17, 2007, p. 3778-3783, Busch-Petersen J. Current Topics in Medicinal Chemistry, 2006, 6, p. 1345-135, Allegretti et al, Immunology Letters 2012, Vol. 145, p. 68-78, Sitaru et al., Internal and Emergency Medicine (2023) 18: 1647-1664).

Preferably, the CXCL8 inhibitor according to the invention is selected from the group or consisting of:
- the anti-CXCL8 antibodies ABCream, BMS-986253 and ABX-IL-8;
- RP-72, PAC-G-31-P, SCH-N,
- Navarixin, having formula: 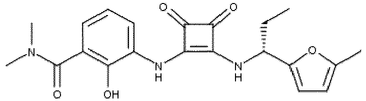
- SX-517, having formula: 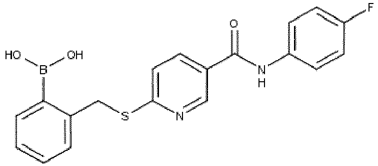
- SX-576, having formula: 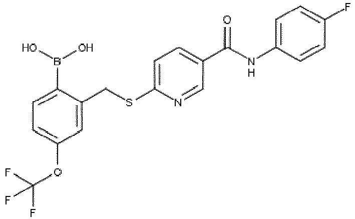
- SX-682 having formula: 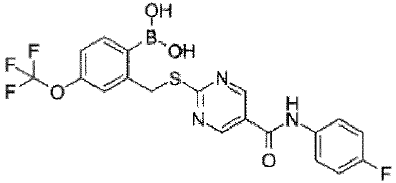
- compounds having formula: 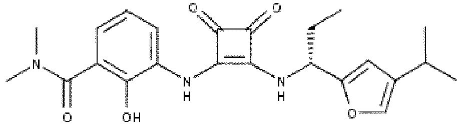 or 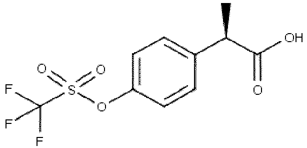 or 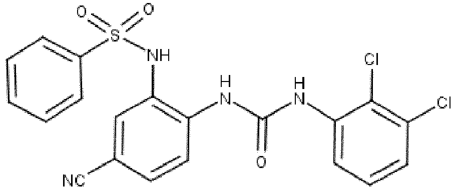 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N, N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) described hereinbelow.

According to one preferred embodiment, said CXCL8 inhibitor has general formula (I): 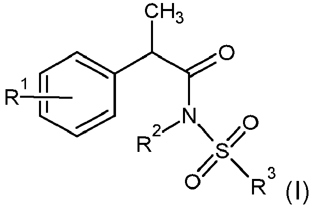 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof.

According to the present invention, "C₁-C₆-alkyl" represents a linear or branched alkyl chain containing 1 to 6 carbon atoms.

R¹ is preferably selected from benzoyl, isobutyl, and trifluoromethanesulfonyloxy. R¹ is preferably linked to the phenyl ring in 3- or 4-position. According to the most preferred embodiment, R¹ is 3-benzoyl, 4-isobutyl or 4-trifluoromethanesulfonyloxy.

R² is preferably selected from hydrogen or methyl.

R³ is preferably selected from linear or branched C₁-C₆ alkyl, more preferably from linear of branched C₁-C₃ alkyl. According to the most preferred embodiment, R³ is methyl.

The chiral carbon of the compounds of formula (I) is in the RS or R configuration, more preferably it is in the R configuration.

Particularly preferred CXCL8 inhibitors compounds of formula (I) according to the invention are selected from:
- 2-(4-isobutylphenyl)propionyl methansulfonamide, preferably R-(-)-2-(4-isobutylphenyl)propionyl methansulfonamide (also known as reparixin) and pharmaceutically acceptable salts thereof, preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide and pharmaceutically acceptable salts thereof, in particular the sodium salt thereof (also known as ladarixin or DF2156A).

Compounds of formula (I) are described in WO2000/024710A1 and WO2005/090295A2, which also disclose their method of synthesis.

According to one preferred embodiment, said CXCL8 inhibitor has general formula (II): 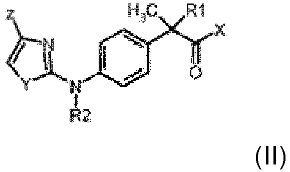 wherein:
R1 is hydrogen or linear or branched C₁-C₄ alkyl, more preferably methyl;
X is OH;
R2 is hydrogen or linear C₁-C₄ alkyl,
Y is a heteroatom selected from S, O and N,
Z is selected from linear or branched C₁-C₄ alkyl, linear or branched C₁-C₄ alkoxy, halo C₁-C₃ alkyl and halo C₁-C₃ alkoxy,
or a pharmaceutically acceptable salt thereof.

Preferably, the chiral carbon of the compounds of formula (II) is in the R or S configuration, more preferably in the S configuration.

Particularly preferred compounds of formula (II) according to the invention are 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof, and 2-methyl-2-(4-{[4-(trifluoromethyl)-1,3-thiazol-2-yl]amino} phenyl)propanoic acid (DF2726Y) and pharmaceutically acceptable salts thereof, preferably the sodium salt (DF2726A).

Compounds of formula (II) are described in WO2010/031835A2, which also discloses their method of synthesis.

Preferred CXCL8 inhibitors according to the invention are DF2726Y and DF2726A, more preferably the CXCL8 inhibitor is DF2726A.

The CXCL8 inhibitor for use according to the present invention may form stable pharmaceutically acceptable salts with a pharmaceutically acceptable organic or inorganic acid or base, and in such cases administration of a compound as a salt may be appropriate.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Non-toxic physiologically-acceptable salts are preferred, although other salts may be useful, such as in isolating or purifying the product.

The salts may be formed by conventional means, such as by reacting the free form of the product with one or more equivalents of the appropriate acid or base in a solvent or medium in which the salt is insoluble, such as for example water or ethanol, which is removed under vacuum or by freeze drying.

The present invention also includes the prodrugs, stereoisomers, isotope-labelled, for example deuterated, derivatives and enantiomers of the CXCL8 inhibitor compounds described above.

As used herein the term "prodrug" refers to an agent, which is converted into the parent drug in vivo by some physiological process (e.g., a prodrug on being brought to the physiological pH is converted to the desired drug form). Prodrugs are often useful because, in some situations, they may be easier to administer than the parent drug. They may, for instance, be bioavailable by oral administration whereas the parent drug is not. The prodrug may also have improved solubility in pharmacological compositions over the parent drug. An example, without limitation, of a prodrug would be a compound of the present invention wherein it is administered as an ester (the "prodrug") to facilitate the transfer across a cell membrane, where water solubility is detrimental, but then it is metabolically hydrolysed once inside the cell where water solubility is beneficial.

Prodrugs have many useful properties. For example, a prodrug may be more water-soluble than the ultimate drug, thereby facilitating intravenous administration of the drug. A prodrug may also have a higher level of oral bioavailability than the ultimate drug. After administration, the prodrug is enzymatically or chemically cleaved to deliver the ultimate drug in the blood or tissue.

Ester prodrugs of the CXCL8 inhibitor compounds disclosed herein are specifically contemplated. While not intending to be limiting, an ester may be an alkyl ester, an aryl ester, or a heteroaryl ester. The term alkyl has the meaning generally understood by those skilled in the art and refers to linear, branched, or cyclic alkyl moieties. C1-6 alkyl esters are particularly useful, where alkyl part of the ester has from 1 to 6 carbon atoms and includes, but is not limited to, methyl, ethyl, propyl, isopropyl, n-butyl, sec-butyl, iso-butyl, t-butyl, pentyl isomers, hexyl isomers, cyclopropyl, cyclobutyl, cyclopentyl, cyclohexyl, and combinations thereof.

Certain CXCL8 inhibitors may exist in tautomeric forms, and this invention includes all such tautomeric forms of those compounds unless otherwise specified.

Unless otherwise stated, structures depicted herein are also meant to include all stereochemical forms of the structure; i.e., the R and S configurations for each asymmetric centre. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as a mixture of such isomers in any ratio.

Particular optical isomers can be obtained by resolution of the racemic mixtures according to conventional processes, e.g., by formation of diastereomeric salts, by treatment with an optically active acid or base or enzymatically. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolysed to deliver the enantiomerically pure compound. Optically active compounds of the invention can be obtained by using active starting materials. These isomers may be in the form of a free acid, a free base, an ester or a salt.

The CXCL8 inhibitors for use according to the present invention may be in amorphous or crystalline form, including any polymorphic form.

According to a preferred embodiment, the CXCL8 inhibitor for use according to the present invention is administered orally.

According to another preferred embodiment, the CXCL8 inhibitor for use according to the present invention is administered by parenteral route, e.g. by intravenous, intraperitoneal, intracerebral, intrathecal, intracranial, intramuscular, intraarticular, intrasynovial, intrasternal, intraocular, intraarterial, subcutaneous, intracutaneous or intralesional injection or infusion techniques.

According to a further preferred embodiment, the CXCL8 inhibitor for use according to the present invention is administered by a route of administration selected from topical, buccal and suppository.

According to a further preferred embodiment, the CXCL8 inhibitor for use according to the present invention is administered by sustained release systems.

The invention is also directed to a pharmaceutical composition comprising at least one CXCL8 inhibitor and at least one pharmaceutically acceptable excipient or carrier for use in the treatment of a tumor with low stromal CAV1 levels.

According to a preferred embodiment, the invention is directed to a pharmaceutical composition comprising at least one CXCL8 inhibitor and at least one pharmaceutically acceptable excipient or carrier for use in the prevention and/or treatment of a metastasis of a tumor with low stromal CAV1 levels.

Preferably, said at least one CXCL8 inhibitor is selected from the CXCL8 inhibitors disclosed above.

Preferably, said pharmaceutically acceptable excipient or carrier as used herein includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions.

Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, osmotic regulators, emulsifiers, sweeteners, colorants, flavourings and the like.

Preferably, the pharmaceutical composition of the present invention is prepared in suitable dosage forms comprising an effective amount of a CXCL8 inhibitor, a pharmaceutically acceptable salt thereof, or a prodrug thereof, and at least one inert pharmaceutically acceptable excipient.

The administration of the pharmaceutical composition of the present invention to a patient is in accordance with known methods and may comprise from one to several oral administrations per day (for example, two times a day (BID) or four times a day (QID)), parenteral administration (including intravenous, intraperitoneal, intracerebral, intrathecal, intracranial, intramuscular, intraarticular, intrasynovial, intrasternal, intraocular, intraarterial, subcutaneous, intracutaneous or intralesional injection or infusion techniques), topical, buccal and suppository administration, or by sustained release systems among other routes of administration.

The amount of a CXCL8 inhibitor or the pharmaceutically acceptable salt thereof in the pharmaceutical composition of the present invention can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

The dosage forms of the pharmaceutical composition of the present invention can be prepared by techniques that are familiar to a pharmaceutical chemist, and comprise mixing, granulation, compression, dissolution, sterilization and the like.

According to a further embodiment, the invention is directed to a method of treating a tumor with low stromal CAV1 levels, which comprises administering an effective amount of one or more CXCL8 inhibitors to a subject in need thereof.

According to a preferred embodiment, the invention is directed to a method of preventing and/or treating a metastasis of a tumor with low stromal CAV1 levels, which comprises administering an effective amount of one or more CXCL8 inhibitors to a subject in need thereof.

Preferably, said one or more CXCL8 inhibitors are selected from the CXCL8 inhibitors disclosed above.

An "effective amount" according to the present invention means an amount sufficient to achieve treatment or prevention of the disease. Determination of the effective amounts is well within the capability of those skilled in the art based upon the achievement of a desired effect. An effective amount will depend on factors including, but not limited to, the weight of a subject and/or the degree of the disease or unwanted condition from which a subject suffers.

According to a preferred embodiment, the invention relates also to the use of at least one CXCL8 inhibitor in the manufacture of a medicament for the treatment of a tumor with low stromal CAV1 levels.

According to a preferred embodiment, the invention relates to the use of at least one CXCL8 inhibitor in the manufacture of a medicament for the prevention and/or treatment of a metastasis of a tumor with low stromal CAV1 levels.

Preferably, said at least one CXCL8 inhibitor is selected from the CXCL8 inhibitors disclosed above.

The terms "treatment" and "prevention" as used herein refer to the eradication/amelioration or prevention/delay in onset, respectively, of the disorder being treated or of one or more of the symptoms associated thereof, notwithstanding the fact that the patient may still be afflicted with the underlying disorder.

The term "tumor with low stromal CAV1 levels" is commonly used in the art to identify a tumor with low levels of CAV1 expression in the stroma. This term has a clear meaning for the skilled person, who is able to unambiguously identify which CAV1 expression levels are classified as "low" and which tumors are encompassed within the definition "tumor with low stromal CAV1 levels". The methods for determining the stromal CAV1 expression levels in tumors and for classifying these levels as "low" are known in the art.

Exemplary methods for determining stromal CAV1 expression levels in tumors and for scoring them are described in Zhao, et al., PLoS ONE 8(3): e59102, 2013, and in Ye, et al., Cancer Management and Research 2020:12 8887-8892.

A preferred method for identifying a tumor with low stromal CAV1 levels comprises the following steps:
a) detecting CAV1 levels in the tumor stroma, and
b) scoring the levels of CAV1 detected in step a) in a scale 0 to 2, wherein a score of 0 or 1 identifies a tumor with low stromal CAV1 levels.

Preferably, said detecting CAV1 levels in the tumor stroma of step a) is carried out by immunostaining the tumor stroma with antibodies directed against CAV1.

Suitable immunostaining techniques are known in the art and are described, for instance, in Goetz et al., Cell 146,148-163, July 8, 2011, and in Pellinen et al., Sci Rep 8, 2338, 2018.

Preferably, the immunostaining is carried out as reported in Witkiewicz, et al., Cancer Biology & Therapy 10:2, 135-143; July 15, 2010. According to this method, CAV1 expression in the tumor stroma is assessed using a standard immunoperoxidase method (Biocare Medical Mach 3 Rabbit HRP-polymer, Concord, CA), using a rabbit polyclonal anti-CAV1 antibody (BD Biosciences, diluted 1:4,000). Briefly, paraffin-embedded blocks containing cancer specimens are fixed in 10% neutral-buffered formalin and processed for immunohistochemical analysis. Tissue sections are de-paraffinized in xylene, re-hydrated in ethanol, re-hydrated with water, and washed in 1% phosphate-buffered saline. Prior to primary antibody application, slides undergo antigen retrieval. Slides are steamed for 30 min in DAKO Target Retrieval Solution pH 6.0 (cat#S1699). Cooled at room temperature for 20 min and washed with TBS buffer 5 times. Sections are then blocked with 3% (v/v) H₂O₂ for 10 min. Primary antibody anti-Cav-1 (BD Biosciences) is applied to slides and incubated for 60 min using a 1/4,000 dilution. The IHC process is finalized with application of suitable immune complexes such as Mach3 Rabbit HRP-Polymer (Biocare Medical cat# M3R531L) and visualized with Dako Liquid DAB⁺ Substrate-Chromogen Solution (DAKO cat# K3468) (diaminobenzidine tetrahydrochloride) for 5 min.

Preferably, said scoring of step b) is carried out as reported in Witkiewicz, et al., Cancer Biology & Therapy 10:2, 135-143; July 15, 2010. Briefly, the staining is scored semiquantitatively as negative (0; no staining), weak (1; either diffuse weak staining or strong staining in less than 30% of stromal cells per core), or strong (2; defined as strong staining in 30% or more of the stromal cells).

Preferably, a tumor with low stromal CAV1 levels according to the present invention is a tumor with a score of 0 or 1, preferably 0, as determined by a method comprising the following steps:
a) detecting CAV1 levels in the tumor stroma by immunostaining the tumor stroma with antibodies directed against CAV1, and
b) scoring the levels of CAV1 detected in step a) in a scale 0 to 2, wherein said score is 0 when no staining is detected in step a), 1 when either a diffuse weak staining or a strong staining in less than 30% of stromal cells per core is detected in step a), 2 when a strong staining in 30% or more of the stromal cells is detected in step a).

Preferably said tumor with low stromal CAV1 levels is a tumor with low stromal CAV1 levels as defined above, selected from the group consisting of breast cancer with low stromal CAV1 levels, preferably triple negative breast cancer with low stromal CAV1 levels, lung cancer with low stromal CAV1 levels, colon cancer with low stromal CAV1 levels, gastric cancer with low stromal CAV1 levels, sarcoma with low stromal CAV1 levels and ovarian cancer with low stromal CAV1 levels.

More preferably, said tumor with low stromal CAV1 levels is breast cancer with low stromal CAV1 levels, even more preferably triple negative breast cancer with low stromal CAV1 levels.

Surprisingly, it has been found that the CXCL8 inhibitor for use according to the invention leads to vascular normalization of the tumor to be treated. This "vascular normalization" is characterized by attenuation of hyperpermeability, increased vascular pericyte coverage, a more normal basement membrane, and a resulting reduction in tumor hypoxia and interstitial fluid pressure. These in turn can lead to an improvement in the delivery and efficacy of exogenously administered therapeutics and in the efficacy of radiotherapy.

Thus, according to a preferred embodiment, the CXCL8 inhibitor for use according to the invention is administered in combination with at least one chemotherapeutic agent.

According to a further preferred embodiment, the CXCL8 inhibitor for use according to the invention is administered in combination with radiotherapy.

According to a further preferred embodiment, the CXCL8 inhibitor for use according to the invention is administered in combination with at least one chemotherapeutic agent and radiotherapy.

Preferably, when said tumor with low stromal CAV1 levels is breast cancer with low stromal CAV1 levels, said at least one chemotherapeutic agent is selected from paclitaxel, anthracyclines such as doxorubicin and epirubicin, 5-fluorouracyl, cyclophosphamide and carboplatin.

Preferably, when said tumor with low stromal CAV1 levels is lung cancer with low stromal CAV1 levels, said at least one chemotherapeutic agent is selected from carboplatin, cisplatin, paclitaxel, docetaxel and etoposide.

Preferably, when said tumor with low stromal CAV1 levels is colon cancer with low stromal CAV1 levels, said at least one chemotherapeutic agent is selected from bevacizumab, irinotechan hydrochloride, capecitabine, oxaliplatin and cetuximab.

Preferably, when said tumor with low stromal CAV1 levels is gastric cancer with low stromal CAV1 levels, said at least one chemotherapeutic agent is selected from docetaxel, doxorubicin and capecitabine.

Preferably, when said tumor with low stromal CAV1 levels is sarcoma with low stromal CAV1 levels, said at least one chemotherapeutic agent is selected from ifosfamide and doxorubicin.

Preferably, when said tumor with low stromal CAV1 levels is ovarian cancer with low stromal CAV1 levels, said at least one chemotherapeutic agent is selected from paclitaxel, altretamine, cyclophosphamide, capecitabine, etoposide and gemcitabine.

Surprisingly, the CXCL8 inhibitors according to the invention led to improved vessel perfusion and decreased metastasis in mice bearing tumors with low stromal CAV1 levels by the inhibition of CXCL8 signalling.

As will be shown in the following experimental section, pharmacological intervention of compounds according to the invention abrogated the alterations and aggressiveness displayed by tumors with low stromal CAV1 levels. These mechanisms allowed to normalize tumor vasculature, decrease tumor aggressiveness and improve intratumor access of other compounds or immune effectors resulting in an improved drug delivery.

The invention is further illustrated by the following examples.

### EXPERIMENTAL SECTION

A study was designed to investigate and show the effect exerted by the compound DF2726A and by a CXCL8 (IL-8) neutralizing antibody on the progression and metastatic potential of a tumor with low stromal CAV1 levels.

For this study, NOD.Cg-*Prkdc^{scid} Il2rg^{tm1wjl}*/SzJ (NOD-SCID IL2rγnull 4; NSG) immunodeficient mice purchased from Jackson Laboratory (Bar Harbor, ME, USA) and housed under specific pathogen-free conditions in accordance with CNIC institutional guidelines were used. Experiments were performed in accordance with Spanish legislation on animal protection and were approved by the local governmental animal care committee. Specifically, young female mice ranging from 8 to 10 weeks old were injected in the fat mammary pad with breast cancer cells.

### EXAMPLE 1

### Materials and methods

### In vivo intervention of CXCL8-driven signalling in xenograft-bearing mice

In order to test the effect of CXCL8 inhibition on primary tumor size, tumor vasculature development and metastatic dissemination, xenograft-bearing mice were subjected to a pharmacological treatment consisting in daily administration throughout 3 weeks of a non-commercial CXCR1/2 antagonist compound (DF2726A). Untreated primary tumor growth post-orthotopic cell injection was allowed for 8 days prior starting drug treatment in order to facilitate engraftment of TCs + CAFs. Afterward, mice were administered daily via oral gavage with 200µl of saline solution containing DF2726A at a dosage of 30 mg/kg/day. Drug solute was resuspended daily prior to administration in order to avoid degradation of the active compound. At day 32 post-cell injection (day 24 after starting the drug treatment) mice were mastectomized to assess primary tumor size, vasculature perfusion (isolectin staining) and blood vessel maturation (PDGFRB staining). 60 days post-mastectomy mice were sacrificed and tissues collected for histological assessment of metastatic dissemination.

### Study design

40 animals were subjected to xenografting of MDA-MB-436 human breast tumor cells (ATCC^{®} HTB-130^{™}, obtained from the American Type Culture Collection (ATCC)) stably expressing luciferase, together with either sCAV1high (having high levels of CAV1) or sCAV1low (having low levels of CAV1) human breast cancer-associated fibroblasts (CAFs).

8 days after xenografting, animals were distributed in 4 groups, 10 animals each, and administered a daily oral (oral gavage) treatment for 21 days as follows:
GROUP 1: sCAV1high CAFs, vehicle administration *(SCRsh CAFs, vehicle).* This group is representative of vehicle treatment of a tumor with high stromal CAV1 levels.
GROUP 2: sCAV1high CAFs, 30mg/kg DF2726A *(SCRsh CAFs, DF2726A batch num. GLB16015).* This group is representative of drug treatment of a tumor with high stromal CAV1 levels.
GROUP 3: sCAV1low CAFs, vehicle administration *(shCAV1 CAFs, vehicle).* This group is representative of vehicle treatment of a tumor with low stromal CAV1 levels.
GROUP 4: sCAV1low CAFs, 30mg/kg DF2726A *(shCAV1 CAFs, DF2726A batch num. GLB16015).* This group is representative of drug treatment of a tumor with low stromal CAV1 levels.

On day 29, treatments were stopped. On day 32, primary tumor size was evaluated after tumor resection (mastectomy).

60 days after tumor resection, animals were sacrificed to harvest samples from primary tumor site, axillary metastases and lung metastases. Lung metastasis was further evaluated by histochemical analysis, as inferred from relative metastasis area. Architecture of stroma and lymph and blood vessel density and arrangement was assessed by confocal sectioning after immunostaining with an endothelial marker (antiCD31 antibody, clone 2H8 (Merck Millipore, Cat. No MAB1398Z)) or lymphatic vessel marker (Anti-LYVE1 Rabbit pAb (Abcam, ab14917)).

### Statistical analysis

Group-group comparisons were subject to unpaired, parametric, two-tailed t-Test analysis. ***:p< 0.005; **:p< 0.01; *p< 0.05; n.s. p> 0.05. To assess statistical significance of differences in event distribution (metastasis) the Barnard's exact test was computed.

### a. Vascular architecture of primary tumors

### a.1 Blood vessel architecture

The obtained results (Fig. 1) support a significant impact of DF2726A exposure on vasculature density and architecture (higher branching/fragmentation as inferred from average distances between contiguous branchpoints, BP-BP) on both genotypes. Exposure to DF2726A abrogated the architectural differences observed between scrCAF tumors and shCAV1 CAF tumors in control conditions; however, this effect appears to be composite between opposite effects on each genotype: trends on reduced BP-BP distance on scrCAFs (sCAV1high CAFs), and increased BP-BP distance on shCAV1 CAFs (sCAV1low CAFs), upon DF2726A exposure.

### a.2 Lymphatic vasculature

The obtained results (Fig. 2) support that DF2726A treatment reduces lymphangiogenesis in the experimental tumor settings in shCAV1 CAF (sCAV1low CAF) tumors, while promoting mild but statistically significant lymphangiogenesis in scrCAF (sCAV1high CAF) tumors. Plot in panel 2B of Fig. 2 represents the percentage of LYVE1-+ area (above 1.4x whole image average) across 3 independent fields per primary tumor (n individuals: 10 per genotype and condition).

### b. Assessment of metastasis

The imbalance between pro- and anti-angiogenic cues found in tumors, a condition that the present study showed to be aggravated in sCAV1low TNBC xenografts, is responsible for the formation of structurally aberrant and dysfunctional tumor vascular networks. This situation, in turn, aggravates tumor hypoxia and promotes a general increase in aggressiveness, traits also displayed by the described sCAV1low tumors.

DF2726A, a dual CXCR1/CXCR2 antagonist, was orally administered to mice during primary tumor growth after xenografting (see Materials and Methods). Upon CXCL8 inhibition, the vasculature of both low and high stromal CAV1 tumor xenografts showed increased pericyte coverage, a phenomenon associated with vascular maturation and stabilization (Fig. 3A). Interestingly, and despite vessel maturation (in terms of increased pericyte coverage) being apparent both in sCAV1high and sCAV1low tumors, the effect of CXCL8 inhibition over blood vessel perfusion showed opposite results when comparing both tumor sets. While sCAV1high xenografts showed decreased perfusion upon CXCL8 inhibition, sCAV1low xenografts displayed an ameliorated blood flow, as assessed by isolectin intravenous injection assays (Fig. 3B and 4). These effects correlated with changes in tumor vascular density (Fig. 1), as well as reduction in lymphatic vessel development (Fig. 2).

Notably, recovered perfusion in sCAV1low tumors upon CXCL8 inhibition was associated with decreased incidence of axillary lymph node metastasis (both upon visual inspection and subsequent histological examination of nodes) (Fig. 5A). The response in sCAV1high tumors had an opposite direction (Fig. 5A). A similar effect was observed regarding lung metastasis incidence rate and lung metastasis relative extension (Fig. 5B and 6).

Based on the above, it can be concluded that tumor blood vessel normalization via CXCL8 receptor inhibition resulted in decreased incidence of metastasis in sCAV1low tumor xenografts. CXCL8 receptor inhibition improved tumor vasculature functionality and decreased aggressiveness in sCAV1 low tumors.

### b.1 Absolute incidence of metastasis

Metastasis incidence after primary tumor resection was assessed by anatomopathological inspection across indicated genotypes and treatments, and it was expressed as % of animals for which metastatic foci were detected in either location (Axillary metastasis - Pulmonary metastasis).

Using Barnard's exact test, a significant effect for exposure to the drug DF2726A on shCAV1 CAF (SCAV1low CAF) tumors was observed when regarding both the metastatic incidence and location (Fig. 5A, 5B).

**Table 1**

| | SCRshCAF | | shCAV1 CAF | |
|---|---|---|---|---|
| | Axillary metastasis | Lung metastasis | Axillary metastasis | Lung metastasis |
| Score statistic | 3.0696 | 2.8867 | -3.08 | -2.886751 |
| Nuisance parameter | 0.0001 | 0.0001 | 0.0001 | 0.0001 |
| p value | <0.0005 | <0.0005 | <0.0005 | <0.0005 |

### b.2 Relative metastasis extension (lung)

The relative extension of metastasis as compared to inspected regions was computed as % of total lung inspected fields, from H-E sections collected from animals for which metastasis had been detected. *: p < 0.05. A statistically significant effect on metastasis extension for drug DF2726A exposure was found on lung samples from animals onto which low stromal CAV1 had been xenografted (Fig. 6).

### Conclusions

Oral supplementation of DF2726A to animals bearing tumors with low stromal CAV1 levels exhibited vascular normalization (Fig. 1) indicating improved perfusion and reduced stress, as suggested by significantly lower development of intratumoral lymphatic vessels (Fig. 2). This was associated with reduced hallmarks of malignancy and metastatic behavior: reduced absolute metastasis incidence at both prime breast tumor metastasis locations (axillary lymph nodes and lung (Fig. 5A, 5B); and reduced relative metastasis extension in the lung (Fig. 6).

Therefore, inhibition of CXCL8 receptor signalling resulted in improved vessel perfusion and decreased metastasis in mice bearing low stromal CAV1 tumors.

### EXAMPLE 2

Xenograft-bearing mice were administered orthotopically with respect to the tumor with a CXCL8 (IL-8) neutralizing monoclonal antibody. Briefly, untreated primary tumor growth post-orthotopic cell inoculation was allowed for 3 days prior starting antibody injections in order to facilitate engraftment of TCs + CAFs. Subsequently, mice were administered every three days (7 injections total) an intratumoral injection of 45µl of saline solution containing 22,5µg of anti-IL-8 mAb (Biotechne, Cat. No MAB208-500). At day 28 post-cell inoculation mice were mastectomized to assess primary tumor size, blood vessel maturation (PDGFRB staining) and lymphangiogenesis (LYVE1 staining). 60 days post-mastectomy mice were sacrificed and tissues collected for histological assessment of metastatic dissemination.

Administration of IL-8-neutralizing antibodies had an analogous effect on tumor blood and lymphatic vascularization (Fig. 7A and B) and metastatic incidence (Fig. 8A and B) as previously observed with DF2726A, albeit in this case a mild reduction in lung metastasis incidence was observed for sCAV1high tumors.

### Conclusions

Taken together, the results of the studies support that CXCL8 dysregulation in tumors with low CAV1 expression in CAFs drives an altered, dysfunctional vascularization, which promotes an aggressive tumor cell behaviour. Intervention of this signalling pathway normalizes vascularization and reduces metastasis incidence specifically in low stromal CAV1 tumors. Thus, the study supports the efficacy of the use of CXCL8 inhibitors for the treatment of tumors with low stromal CAV1 levels and for the prevention/treatment of metastases of tumors with low stromal CAV1 levels.

## Claims

1. A CXCL8 inhibitor for use in the treatment of a tumor with low stromal CAV1 levels.

2. A CXCL8 inhibitor for use according to claim 1, wherein said CXCL8 inhibitor is for use in the prevention and/or treatment of a metastasis of said tumor with low stromal CAV1 levels.

3. A CXCL8 inhibitor for use according to claim 1 or claim 2, which is a CXCL8 receptor(s) inhibitor selected from a CXCR1- or a CXCR1/2-inhibitor.

4. A CXCL8 inhibitor for use according to any one of claims 1 to 3, wherein said tumor with low stromal CAV1 levels is a tumor with a score of 0 or 1, preferably 0, as determined by a method comprising the following steps:
a) detecting CAV1 levels in the tumor stroma by immunostaining the tumor stroma with antibodies directed against CAV1, and
b) scoring the levels of CAV1 detected in step a) in a scale 0 to 2, wherein said score is 0 when no staining is detected in step a), 1 when either a diffuse weak staining or a strong staining in less than 30% of stromal cells per core is detected in step a), 2 when a strong staining in 30% or more of the stromal cells is detected in step a).

5. A CXCL8 inhibitor for use according to any one of claims 1 to 4, wherein said inhibitor is selected from the group consisting of:
- the anti-CXCL-8 antibodies ABCream, BMS-986253 and ABX-IL-8;
- RP-72, PAC-G-31-P, SCH-N,
- Navarixin, having formula: 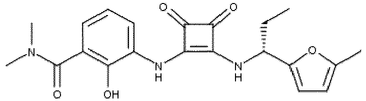
- SX-517, having formula: 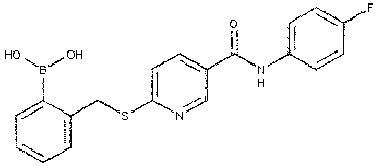
- SX-576, having formula: 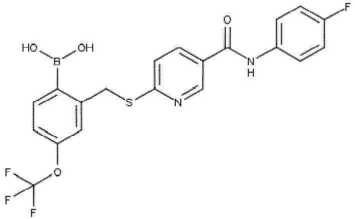
- SX-682 having formula: 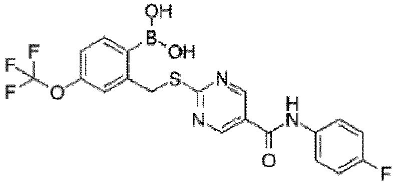
- compounds having formula: 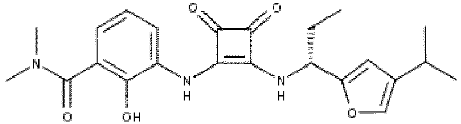 or 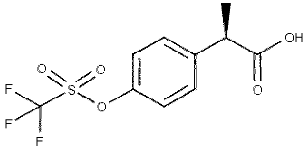 or 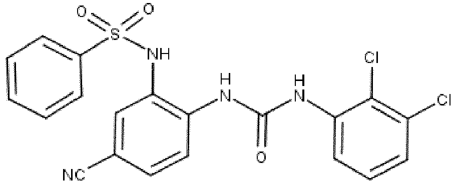 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide;
- a compound having general formula (I): 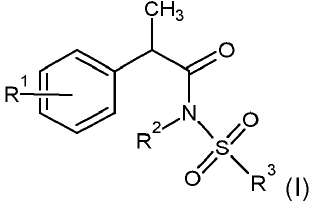 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof; and
- a compound having general formula (II): 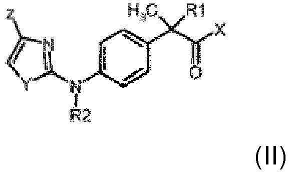 wherein:
R1 is hydrogen or linear or branched C₁-C₄ alkyl;
X is OH;
R2 is hydrogen or linear C₁-C₄ alkyl,
Y is a heteroatom selected from S, O and N,
Z is selected from linear or branched C₁-C₄ alkyl, linear or branched C₁-C₄ alkoxy, halo C₁-C₃ alkyl and halo C₁-C₃ alkoxy,
or pharmaceutically acceptable salts thereof.

6. A CXCL8 inhibitor for use according to claim 5, wherein the chiral carbon of the compounds of formula (I) is in the R configuration.

7. A CXCL8 inhibitor for use according to claim 5, wherein the chiral carbon of the compounds of formula (II) is in the S configuration.

8. A CXCL8 inhibitor for use according to claim 5 or 6, having general formula (I) which is selected from:
- 2-(4-isobutylphenyl)propionyl methansulfonamide, preferably R-(-)-2-(4-isobutylphenyl)propionyl methansulfonamide and pharmaceutically acceptable salts thereof, preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide and pharmaceutically acceptable salts thereof, in particular the sodium salt thereof.

9. A CXCL8 inhibitor for use according to claim 5 or 7, having general formula (II) which is selected from 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof and 2-methyl-2-(4-{[4-(trifluoromethyl)-1,3-thiazol-2-yl]amino} phenyl)propanoic acid and pharmaceutically acceptable salts thereof, preferably the sodium salt.

10. A CXCL8 inhibitor for use according to claim 9, which is the sodium salt of 2-methyl-2-(4-{[4-(trifluoromethyl)-1,3-thiazol-2-yl]amino} phenyl)propanoic acid.

11. A CXCL8 inhibitor for use according to any one of claims 1 to 10, wherein said tumor with low stromal CAV1 levels is selected from the group consisting of breast cancer with low stromal CAV1 levels, preferably triple negative breast cancer with low stromal CAV1 levels, lung cancer with low stromal CAV1 levels, colon cancer with low stromal CAV1 levels, sarcoma with low stromal CAV1 levels, gastric cancer with low stromal CAV1 levels and ovarian cancer with low stromal CAV1 levels.

12. A CXCL8 inhibitor for use according to any one of claims 1 to 11, wherein said CXCL8 inhibitor is administered in combination with at least one chemotherapeutic agent and/or radiotherapy.

13. A CXCL8 inhibitor for use according to claim 12, wherein said tumor with low stromal CAV1 levels is breast cancer with low stromal CAV1 levels and said at least one chemotherapeutic agent is selected from paclitaxel, anthracyclines such as doxorubicin and epirubicin, 5-fluorouracyl, cyclophosphamide and carboplatin, or wherein said tumor with low stromal CAV1 levels is lung cancer with low stromal CAV1 levels and said at least one chemotherapeutic agent is selected from carboplatin, cisplatin, paclitaxel, docetaxel and etoposide, or wherein said tumor with low stromal CAV1 levels is colon cancer with low stromal CAV1 levels and said at least one chemotherapeutic agent is selected from bevacizumab, irinotechan hydrochloride, capecitabine, oxaliplatin and cetuximab, or wherein said tumor with low stromal CAV1 levels is sarcoma with low stromal CAV1 levels and said at least one chemotherapeutic agent is selected from ifosfamide and doxorubicin, or wherein said tumor with low stromal CAV1 levels is ovarian cancer with low stromal CAV1 levels and said at least one chemotherapeutic agent is selected from paclitaxel, altretamine, cyclophosphamide, capecitabine, etoposide and gemcitabine, or wherein said tumor with low stromal CAV1 levels is gastric cancer with low stromal CAV1 levels and said at least one chemotherapeutic agent is selected from docetaxel, doxorubicin and capecitabine.
